# EUROPEAN PATENT SPECIFICATION

(11) **EP 0 462 123 B1**
(45) Date of publication and mention of the grant of the patent: **24.09.1997**
(21) Application number: 90903716.0
(22) Date of filing: 23.02.1990
(51) Int. Cl.: C07D 211/70, C07D 221/20, A61K 7/42, C09D 5/32, C08L 23/12, C08K 5/00

(54) **UV-ABSORBING COMPOUNDS AND COMPOSITIONS**
UV-ABSORBIERENDE VERBINDUNGEN UND ZUSAMMENSETZUNGEN
COMPOSES ET COMPOSITIONS ABSORBANT LES UV

(30) Priority: 23.02.1989 AU 2891/89; 23.02.1989 AU 2892/89
(43) Date of publication of application: 27.12.1991
(73) Proprietor: AUSTRALIAN INSTITUTE OF MARINE SCIENCE, Cleveland, QLD 4810 (AU)
(72) Inventor: CHALMERS, Peter, James, Sunbury, VIC 3429 (AU); FITZMAURICE, Neil, Malvern East, VIC 3144 (AU); RIGG, David, John, Hawthorn East, VIC 3123 (AU); THANG, San, Hoa, Clayton South, VIC 3169 (AU); BIRD, Graham, Ascot Vale, VIC 3032 (AU)
(74) Representative: Andrae, Steffen, Dr.
(86) International application number: AU9000078
(87) International publication number: WO9009995

(56) References cited:
- AU-B- 8 072 887
- TETRAHEDRON LETTERS, no. 46, 1971, pages 4375-4378, Pergamon Press, GB; W.H. DALY et al.: "Deuterium exchange between enamines and hexadeuteroacetone"

## Description

The invention relates to Ultraviolet light absorbing compounds processes for preparation thereof and compositions comprising same.

Ultraviolet light absorbing compounds are useful in protecting a range of materials from deleterious effects caused by exposure to the sun. They are used, for example, to protect skin from erythema edema and blistering and to stabilise compositions such as plastics and paint compositions.

Our copending PCT Application No PCT/AU87/00330 describes a class of UV-absorbing compounds which have a cyclic enaminoketone structure.

While such compounds have proved to be useful as UV-absorbing agents there is a need for compounds of this type which combine the features of high absorption in the appropriate region of the spectrum and have a long storage life.

Accordingly we provide a compound of formula I 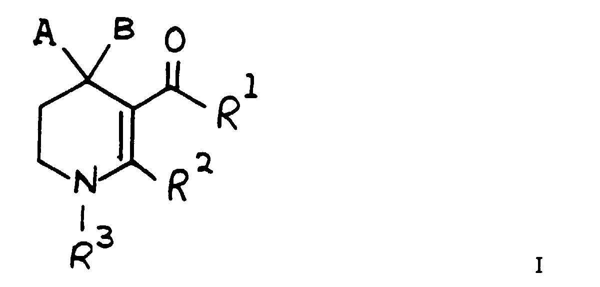 wherein R¹ is selected from the group consisting of C₁ to C₁₈ alkyl; C₁ to C₆ haloalkyl; C₁ to C₁₂ alkyl substituted with a substituent selected from hydroxy, amino, C₁ to C₁₆ alkoxy, C₁ to C₁₆ alkanoyl, (C₁ to C₁₆ alkoxy)-carbonyl, phenyl and the radical of formula I(a) 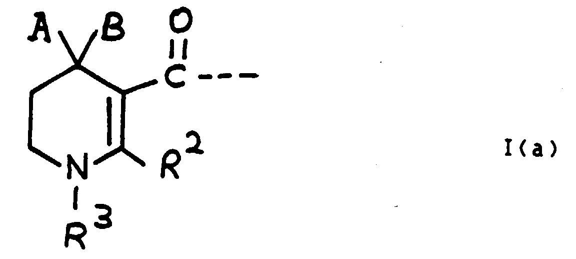 wherein
A, B, R² and R³ are as herein defined; C₂ to C₁₈ alkenyl; C₅ to C₇ Cycloalkyl; phenyl; the groups phenyl and benzyl said groups being substituted in the benzene ring with a substituent selected from C₁ to C₉ alkyl, C₂ to C₉ alkenyl and C₁ to C₉ alkoxy; and wherein R¹ and R² may form a carbocyclic ring by the bridging group of formula - CH₂(R⁷R⁸C)CH₂ - wherein R⁷ and R⁸ are independently selected from hydrogen and C¹ to C⁶ alkyl;
R³ is selected from the group consisting of C₁ to C₁₈ alkyl; C₁ to C₁₂ alkyl substituted with a substituent selected from the group consisting of hydroxy, amino, C₁ to C₁₆ alkoxy, phenyl and C₁ to C₁₆ alkanoyl; C₂ to C₁₈ alkenyl; C₅ to C₇ cycloalkyl; substituted benzyl wherein the benzene ring is substituted with a substituent selected from C₁ to C₉ alkyl, C₂ to C₉ alkenyl and C₁ and C₉ alkoxy;
R² is selected from the group consisting of hydrogen and C₁ to C₄ alkyl
A and B are independently selected from C₁ to C₆ alkyl and C₁ to C₆ haloakyl and may together form a carbocyclic ring of from 3 to 6 ring members.

Preferred R¹ include C₁ to C₁₈ alkyl; C₁ to C₆ haloalkyl; C₁ to C₆ alkyl substituted by a substituent selected from the group consisting of C₁ to C₉ alkoxy, C₁ to C₉ alkanoyl, (C₁ to C₉ alkoxy)-carbonyl and the radical of formula I(a).

More preferably R¹ is C₁ to C₁₈ alkyl or C₁ to C₄ haloakyl. Specific examples of R include methyl, ethyl, n-propyl, 1-methylethyl, 1-ethylpropyl, 2-ethylpropyl, butyl, 1-methelbutyl, 2-methylbutyl, 3-methylbutyl, 3, 3-dimethylbutyl, 1,1-dimethylbutyl, 2,3-dimethylbutyl, pentyl, 1-methylpentyl, trifluoromethyl and pentafluoroethyl, hexyl, heptyl, nonyl, decanyl, tridecanyl, tetradecanyl, pentadecanyl.

Preferred R² is hydrogen.

Preferred R³ include C₁ to C₁₈ alkyl and more preferably C₂ to C₁₈ alkyl. Specific examples of R³ include ethyl, propyl, 1-methylethyl, 1-methylpropyl, 2-methylpropyl, butyl, 1-methylbutyl, 2-methylbutyl, 3-methylbutyl, 1,1-dimethylbutyl, 3,3-dimethylbutyl, pentyl, 2-methylpentyl, hexyl, heptyl, 2-ethylhexyl, octyl, decyl and tetradecyl.

Preferably A and B are independently selected from C₁ to C₄ alkyl and most preferably A and B are methyl.

Although compounds of formula I may be used in a range of applications such as plastic and paint formulations they are particularly suited to use as UV absorbing agents in sunscreen formulations for topical application to human skin. Commercial sunscreen agents are generally preferred to have a high water resistance so that a practically useful period of protection against solar radiation is provided despite immersion in water and the presence of perspiration.

We have found that compounds of the invention comprising from 14 to 31 carbon atoms provide a level of water resistance comparable with, and in many cases superior to the leading commercially available sunscreen agents. Compounds of formula I comprising from 16 to 22 carbon atoms are particularly preferred. We have found that such compounds have the optimum balance of high water resistance and efficient ultra violet radiation absorption.

Water resistance of compounds of formula I may be evaluated by reference to the partition coefficient of the compounds using a non-polar solvent such as octanol in conjunction with water. The term log P, where P is the partition coefficient, where used herein refers to the log P value determined by the HPLC method of David E Leahy, Peter J Taylor and Alan R Wait, Ouant. Struct.-Act Relat., 8, 17-31 (1989) at pages 23 and 24. Log P may be used to directly compare the lipophilicity of a variety of molecules as described by Hansch, C., and Leo, A. J., Substituent Constants for Correlation Analysis in Chemistry and Biology, John Wiley, New York 1979.

It is preferred that in the compounds of formula I the group R³ is C₂ to C₁₈ alkyl. Compounds of formula I wherein R³ is C₂ to C₁₈ alkyl have generally been found to be more stable than their methyl substituted counterparts and generally more convenient to prepare via the intermediate of formula VII.

We thus further provide a compound of formula VII wherein
R² is as herein before defined and R³ is C₂ to C₁₈ alkyl.

Preferably compounds of the invention have a log P in the range of from 3.2 to 10.9. We have found that compounds in this range generally have a good balance of absorbance and water resistance properties.

In order to provide the correct balance of absorbance and water resistance it is particularly preferred that one of the groups R¹ and R³ be selected from C₁ to C₆ alkyl (preferably C₁ to C₄ alkyl) and the other is C₅ to C₁₈ alkyl.

Specific examples of compound of the invention are listed in Table 1 below.

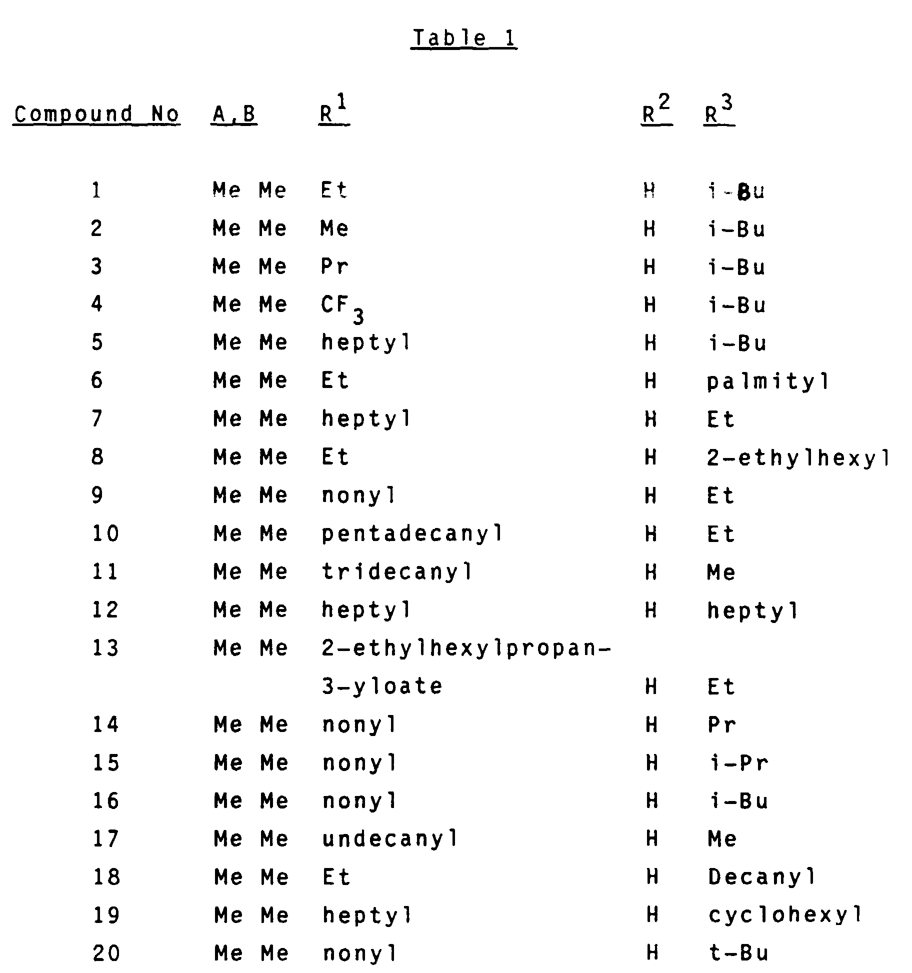

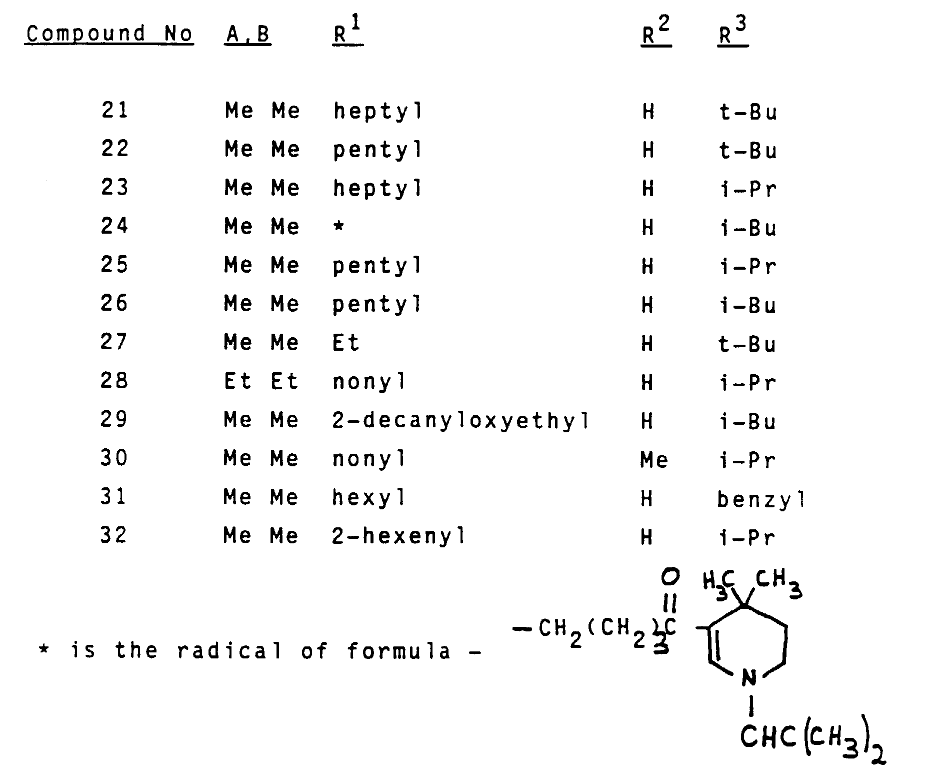

In the above table the following common abbreviations are used:

| | |
|---|---|
| Me is methyl | i-Pr is iso-propyl |
| Et is ethyl | i-Bu is iso-butyl |
| Pr is propyl | t-Bu is tert-butyl |
| Bu is butyl | |

The compounds of the invention may be prepared by a variety of methods.

It is preferred that the compounds of the invention are prepared by reaction of a compound of formula VII with an acylating agent of formula VIII or IX.

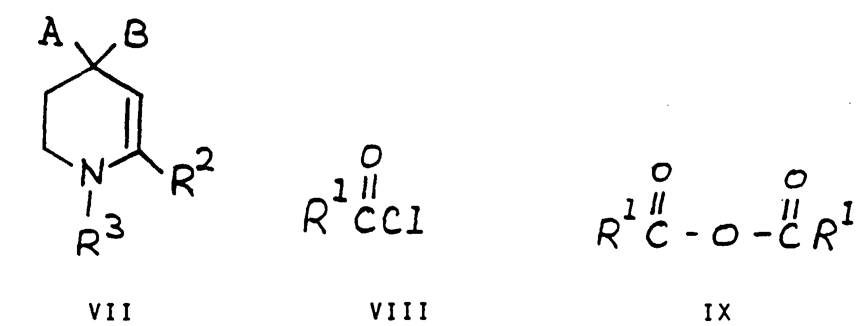

Compounds of formula VII wherein R³ is C₂ to C₁₈ alkyl have good stability and are particulary suited to preparation of compounds of formula I. We further provide a compound of formula VII wherein R³ is C₂ to C₁₈ alkyl and R¹, R², A and B are as hereinbefore defined.

We have found that the intermediate compounds of formula VII may be conveniently prepared in good yield by reaction of the appropriate substituted amino compound with a suitable α-pyrone or substituted pentanoic acid derivative.

Thus we further provide a process for preparing said compounds of formula VII by reacting an amine of formula II with a compound of formula III in order to obtain a compound of formula VII wherein R² is hydrogen; 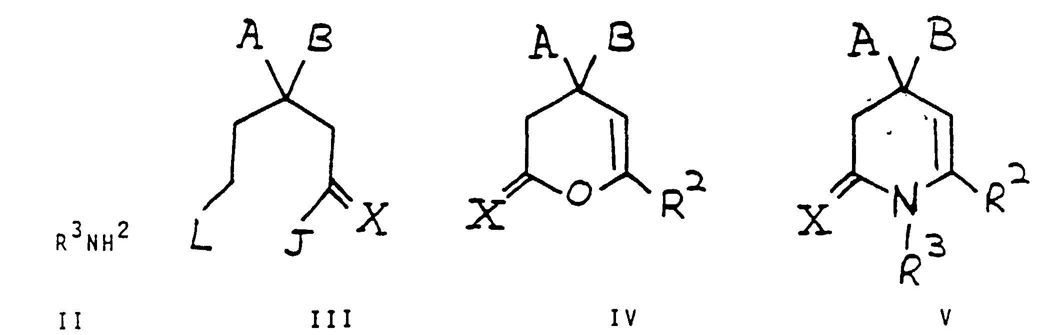 or by reacting an amine of formula II with an α-pyrone of formula IV to provide an α-pyridone of formula V and reducing the α-pyridone; to provide the compound of formula VII. In the compounds of formulae III, IV and V the group X is oxo or a derivative thereof such as an acetal derative.

In the compound of formula III the group L is leaving group and J is selected from the group consisting of hydrogen, hydroxy, chloro and alkoxy. Preferably J is hydrogen, hydroxy or C₁ to C₆ alkoxy and most preferably J is hydrogen.

Examples of leaving groups may include alkylsulfonyl, chlorine, bromine or iodine, mesylate, tosylate, arenesulphonylates and the group where L forms an oxirane, that is, where the compound of formula III has the formula III(a)

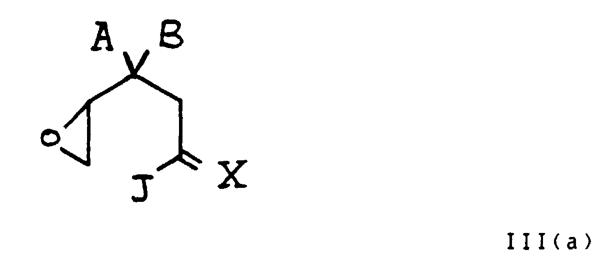

Preferably L is bromine or L forms an oxirane to provide the compound of formula III(a).

Preferred X are oxo, di-(C₁ to C₄ alkoxy), ethylenedioxy, propylenedioxy and 2,2-dimethylpropylenedioxy.

When X is oxo J is typically hydrogen, hydroxy or alkoxy and when X is a derivative of oxo such as an acetal derivative then J is typically hydrogen.

Specific examples of compounds of formula III include the following:

| L | A,B | X | J |
|---|---|---|---|
| Br | Me Me | O | H |
| Br | Me Me | di-OEt | H |
| Br | Me Me | -O-CH₂CH₂-O- | H |
| OTs | Me Me | O | H |
| Br | Me Me | O | OCH₃ |
| Br | Me Me | O | Cl |
| Br | Me Me | -OCH₂C(CH₃)₂CH₂O- | H |
| Br | Me Me | -O(CH₂)₃O- | H |
| Note: OTs is tosylate | | | |

Compounds of formula III are believed to be novel compounds, hence, in a further embodiment of the invention we provide compounds of formula III. 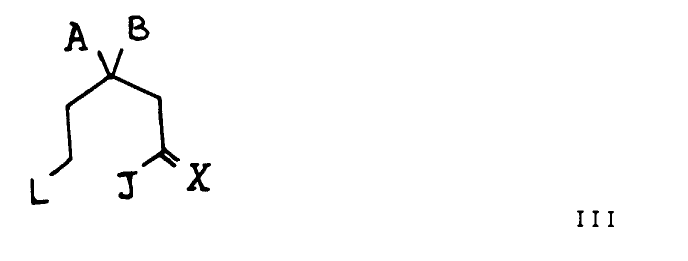 wherein L, A, B, J and X are as hereinbefore defined.

The compound of formula III may be prepared by substitution of an olefin of formula XIII.

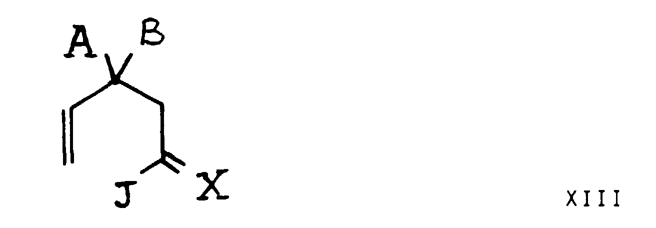

For example, compounds of formula III where L is Bromine may be prepared by reaction of compounds of formula XIII with gaseous hydrogen bromide in the presence of a free radical initiator (anti-Markovniknov).

The compounds of the invention may also be prepared by a method involving the following steps:-
(a) Reacting a glutaric anhydride derivative of formula XI with an amine of formula II to form an imide of formula XII. 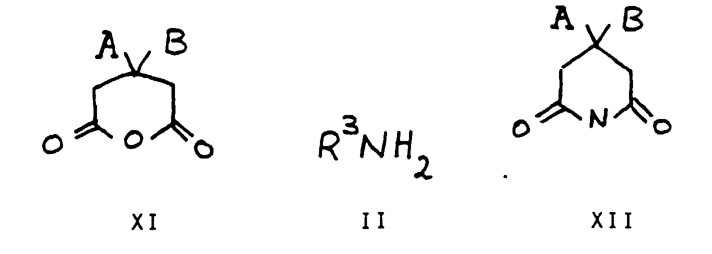
(b) reducing the imide of formula XII using a suitable reducing agent such as lithium aluminium hydride to give a cyclic amine of formula VI. 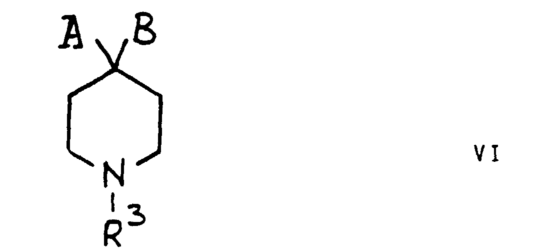
(c) Oxidising the amine of formula VI to form the enamine of formula VII wherein R² is H. The preferred oxidizing agent for this reaction is mercuric acetate. 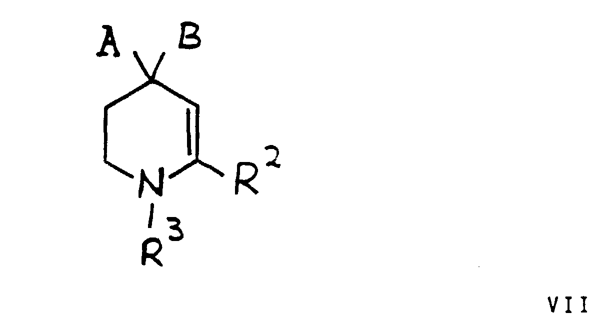
(d) Acylating the compound of formula VII using an acyl chloride of formula VIII or anhydride of formula IX to provide the compound of the invention of formula I.

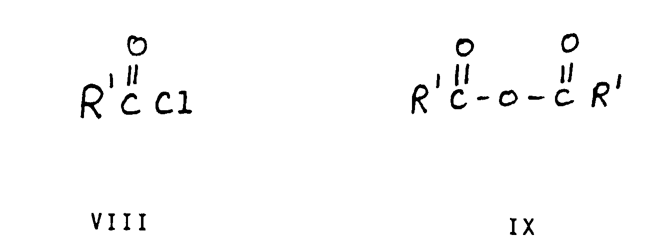

One or more compounds of the invention may be used to reduce the exposure of a surface such as skin to ultravoilet radiation by topical application thereto.

The invention further provides an ultraviolet light absorbing composition comprising as an effective component thereof at least one compound of formula 1.

The level of the UV-absorbing agent of the invention in the compositions is preferably in the range of from 0.001 to 30% w/w of the total composition although the preferred concentration will of course depend on the nature of the composition and the degree of screening required.

In one embodiment of the invention the UV-absorbing composition is a sunscreen composition for topical application to a surface such as skin. Such compositions may be in a variety of forms such as for example solids, liquids, gels or aerosols and will generally comprise a carrier which adapts the agent for topical application.

Examples of suitable carriers may include oils, for example mineral oils, paraffin, squaline and octyl palmitate and oil/alcohol mixtures.

In one embodiment the sunscreen composition is an emulsion such as a oil-in-water emulsion which may for example comprise a water-insoluble liquid (eg paraffin oil) as the oil phase.

Further components such as perfumes, colouring agents, antioxidants and oxygen scavengers may be used in the composition of the invention. The composition may comprise additives making the composition useful as a cosmetic or topical pharmaceutical.

Typically the compounds of the invention will constitute in the range of 0.5 to 30% (preferably 1.0 to 15%) by weight of the total sunscreen composition. However higher or lower concentrations may be used if desired depending on the degree of screening required.

In a further embodiment there is provided a method of preparing a sunscreen composition comprising mixing at least one compound of formula I with a carrier suitably adapted to allow application of said compound to a surface. The temperature at which the composition is prepared may vary depending on the components but is generally in the range of from 5 to 100°C.

In another embodiment of the invention the UV-absorbing composition is a coating composition such as a paint composition, preferably comprising in the range of from 0.001 to 5% w/w of at least one compound of formula I. Typically a paint composition will comprise a binder or film forming component such as alkyd resin, acrylic latex or urethane and may comprise a pigment to provide colouration and/or other optional additives such as fungicides, flow control agents and rheology modifiers.

In another embodiment we provide a plastics composition comprising at least one UV-absorbing agent of formula I.

The base plastic may be any plastic in which protection against UV-induced degradation is desired. The plastic composition will generally comprise a polymeric component and in the range of 0.01 to 45% w/w of the UV-absorbing agent component and may comprise other components, such as one or more of fillers, antioxidants and lubricants, which are known in the art.

The invention will now be described but is in no way limited to the following Examples.

### Example 1

(a) Preparation of 5 Bromo-3,3-dimethylpentanal
   1,1-Diethoxy-3,3-dimethylpent-4-ene was prepared from 3,3-dimethylpent-4-enal, ethanol and hydrogen chloride gas by the method used for the preparation of its dimethoxy analogue derivative described in the literature (Boeckman and Ko, J. Am. Chem. Soc., 104, 1982, p. 1033). ¹H NMR (CDCl₃) δ 5.81 (1H, dd, J 10, 18 H_{z}), 4.90 (1H. dd, J 2.0, 18.0 H_{z}), 4.89 (1H, dd, J 2.0, 10.0 H_{z}), 4.48 (1H, t, J 5.0 H_{z}), 3.55 (2H, q, J 7.0 H_{z}), 3.47 (2H, q, J 7.0 H_{z}), 1.64 (2H, d, J 5.0 H_{z}), 1.18 (6H, t, J 7.0 H_{z}), and 1.04 (6H, s).
(b) Hydrogen bromide gas was bubbled through a solution of benzoyl peroxide (0.1g) (or α, ^{α 1}-azobisisobutyronitrile, 0.1g) and 1,1-diethoxy-3,3-dimethylpent-4-ene in dry pentane (50 ml) stirring under nitrogen at 0-5°C.
   The reaction was monitored by T.L.C. (1:1 dichloromethane: hexane as eluent) until all starting material was consumed (detected by T.L.C. as the 2,4 DNP derivatives of the corresponding aldehydes). Water (20 ml) was added, the mixture stirred vigorously for 5 minutes and the organic layer was separated and the aqueous layer extracted with further pentane (20 ml). The combined organic layer was washed with water (20 ml), dried over magnesium sulphate and concentrated to give 5-bromo-3,3-dimethyl pentanal (19.0g) as a pale yellow oil. Distillation under reduced pressure gave a colourless oil (17.0g, 88%) (b. pt 48-50°C at 0.4mbar. ¹H NMR (CDCl₃) δ 9.77 (1H, t, J2.OH_{z}), 3.39 (2H, m), 2.32 (2H, d, J2.OH_{z}), 1.97 (2H, m), 1.11 (6H, s).
(c) 1-(2-Methylpropyl)-4,4-dimethyl-1,4,5,6-tetrahydropyridine
   Acetic acid was added to a mixture of ethanol (40 ml) and water (20 ml) until the pH was 4. 5-Bromo-3,3-dimethylpentanal (19.3g, 0.1 mol) was added to the mixture, which was then cooled to 0-5°C and purged with nitrogen. Isobutylamine (7.3g, 0.1 mol) was added over 5 minutes to the solution stirring under an atmosphere of nitrogen. After 3 hours stirring at 0-5°C, most of the ethanol was removed under reduced pressure, the residue then diluted with water (20 ml) and the solution taken to pH 10 with solid potassium carbonate. The mixture was extracted with ether (3 x 50 ml), the organic layer dried over anhydrous MgSO₄ and concentrated to yield a pale yellow oil (15.5g, 93%) which was essentially pure by ¹H NMR spectroscopy. A pure colourless liquid (13.0g, 78%) was obtained after vacuum distillation, b.p. 50-53°C/2mm Hg. The ¹H NMR spectrum was found to be the same as for material prepared by the procedure hereafter described in Example 25 Part (C).
(d) I-(2-Methylpropyl)-3-propionyl-4,4-dimethyl-1,4,5,6-tetrahydropyridine
   Propionyl chloride (9.25g, 0.1 mol) was added dropwise to a solution of 1-(2-methylpropyl)-4,4-dimethyl-1,4,5,6-tetra-hydropyridine (16.7g, 0.1 mol) and triethylamine (12.1g, 0.12 mol) in dichloromethane (100 ml) stirring under an atmosphere of nitrogen at ice bath temperature. After one hour at this temperature, water (50 ml) was added, the organic layer was separated, washed with 5% aqueous sodium carbonate and dried over anhydrous MgSO₄. Removal of the organic solvent afforded a yellow oil (22.3g, quantitative) which was essentially pure by 90 MHz NMR spectroscopy. After column chromatography on silica-gel (Merck Kieselgel 60, 70-230 mesh) using 5% diethyl ether in dichloromethane (or 3:7 ether: n-hexane) as eluent, the pure 1-(2-methylpropyl)-3 -propionyl-4,4-dimethyl-1,4,5,6-tetrahydropyridine (18.0g, 81%) was obtained as a very pale yellow oil. UV λ max MeOH 307 nm, 29,300. ¹H NMR (CDCl₃) δ 7.13 (1H, s), 3.10 (2H, m, AA¹XX^{I} system), 2.94 (2H, d, J7.4 H_{z}), 2.44 (2H, q, J7.5 H_{z}), 1.95 (1H, m, J6.6 H_{z}, J7.4 H_{z}), 1.60 (2H, m, AA^{I}XX¹ system), 1.27 (6H, s), 1.08 (3H, t, J7.5 J_{z}), and 0.90 (6H, d, J6.6 H_{z}). ¹³C NMR (CDCl₃) δ 195.9 (s), 147.9 (d), 114,4 (s), 64.1 (t), 43.3 (t), 39.3 (t), 30.0 (s), 29.7 (t), 28.1 (q), 27.4 (d), 19.8 (q), and 10.3 (q). MS 223.1913 (M⁺), 25.2%, 194 (100%).

### Examples A to J

The compounds of these examples were prepared by the method of Example 1 with the appropriate amine shown in the table 2 below being substituted for isobutylamine in Example 1 part (c).

**Table 2**

| Example | Amine | Product Name |
|---|---|---|
| A | palmitylamine | 1-palmityl-4,4-dimethyl-1,4, 5,6-tetrahydropyridine |
| | | |
| B | ethylamine | 1-ethyl-4,4-dimethyl-1,4,5,6-tetrahydropyridine |
| | | |
| C | 2-ethylhexylamine | 1-(2-ethylhexyl)-4,4-dimethyl-1,4,5,6-tetrahydropyridine. |
| | | |
| D | methylamine | 1,4,4-trimethyl-1,4,5,6-tetrahydropyridine |
| | | |
| E | heptylamine | 1-heptyl-4,4-dimethyl-1,4,5,6-tetrahydropyridine |
| | | |
| F | isopropylamine | 1-(1-methylethyl)-4,4-dimethyl-1,4,5,6-tetrahydro pyridine |
| | | |
| G | propylamine | I-propyl-4,4-dimethyl-1,4,5,6-tetrahydropyridine |
| | | |
| H | decylamine | 1-decyl-4,4-dimethyl-1,4,5,6-tetrahydropyridine |
| | | |
| I | cyclohexylamine | 1-cyclohexyl-4,4-dimethyl-1,4,5,6-tetrahydropyridine |
| | | |
| J | tert-butylamine | 1-(1,1-dimethylethyl)-4,4-dimethyl-1,4,5,6-tetrahydropyridine |

### Examples 2 to 23

The compounds of examples 2 to 23 were prepared by reaction of the corresponding tetrahydropyridine intermediate identified in table 4 with the appropriate acid chloride identified in the table 4 using the procedure of Example 1 to provide the product as detailed in Table 4.

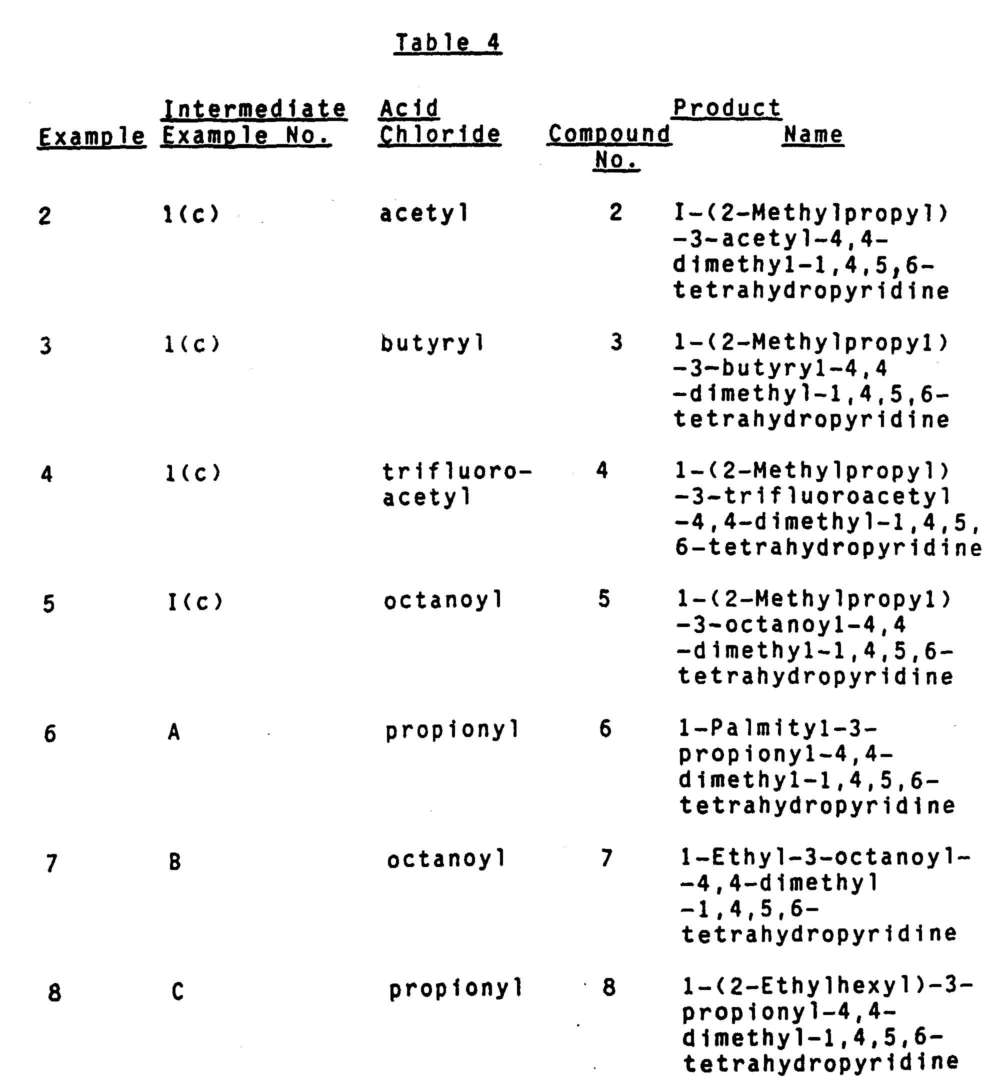

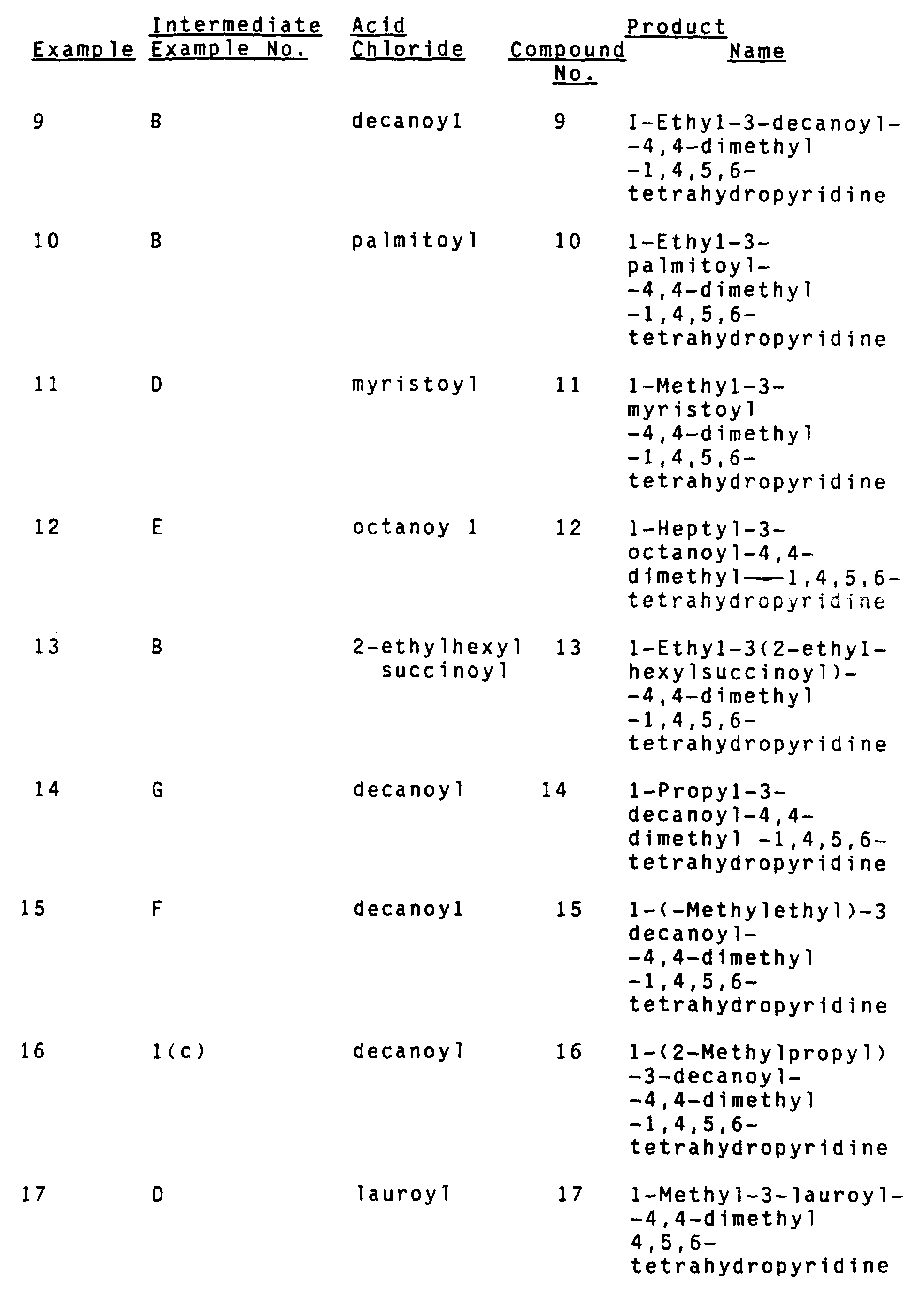

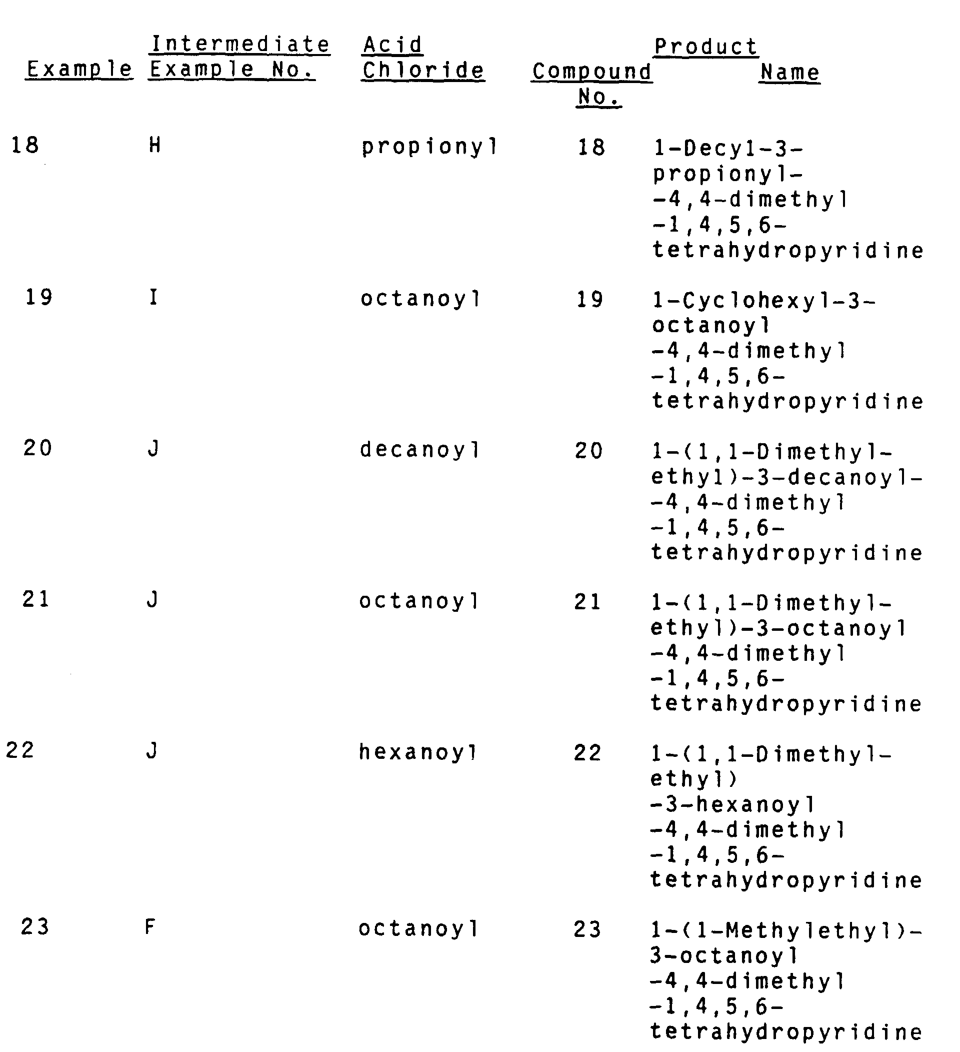

The proton nuclear magnetic resonance spectra and ultra violet spectra of compounds 1 to 23 were measured and are recorded in Table 5 (except where indicated by a hyphen).

### Example 24

### 1,1'- (2-Methylpropyl)-3,3'-adipoyl-4,4'-dimethyl-bis (1,4,5,6-tetrahydropyridine) (24)

The title compound was prepared in accordance with the procedure of Example 1 Part (C) replacing propionyl chloride with adipoyl chloride with the exception that, due to the stoicheiometry of the reaction, an approximate molar ratio of adipoyl chloride: 1-(2-Methylpropyl)-4, 4-dimethyl-1,4,5,6-tetrahydropyridine of 1:2 was used. The proton nuclear magnetic responance spectrum and ultra violet spectrum of the product was measured and is recorded in Table 5.

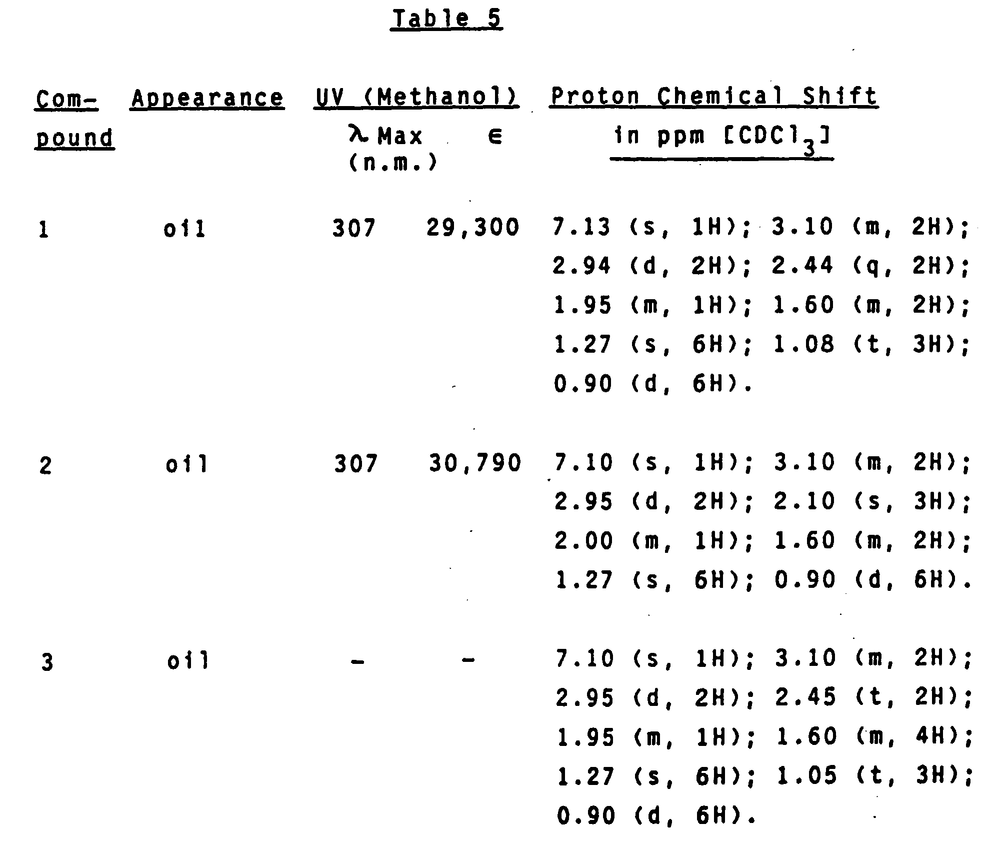

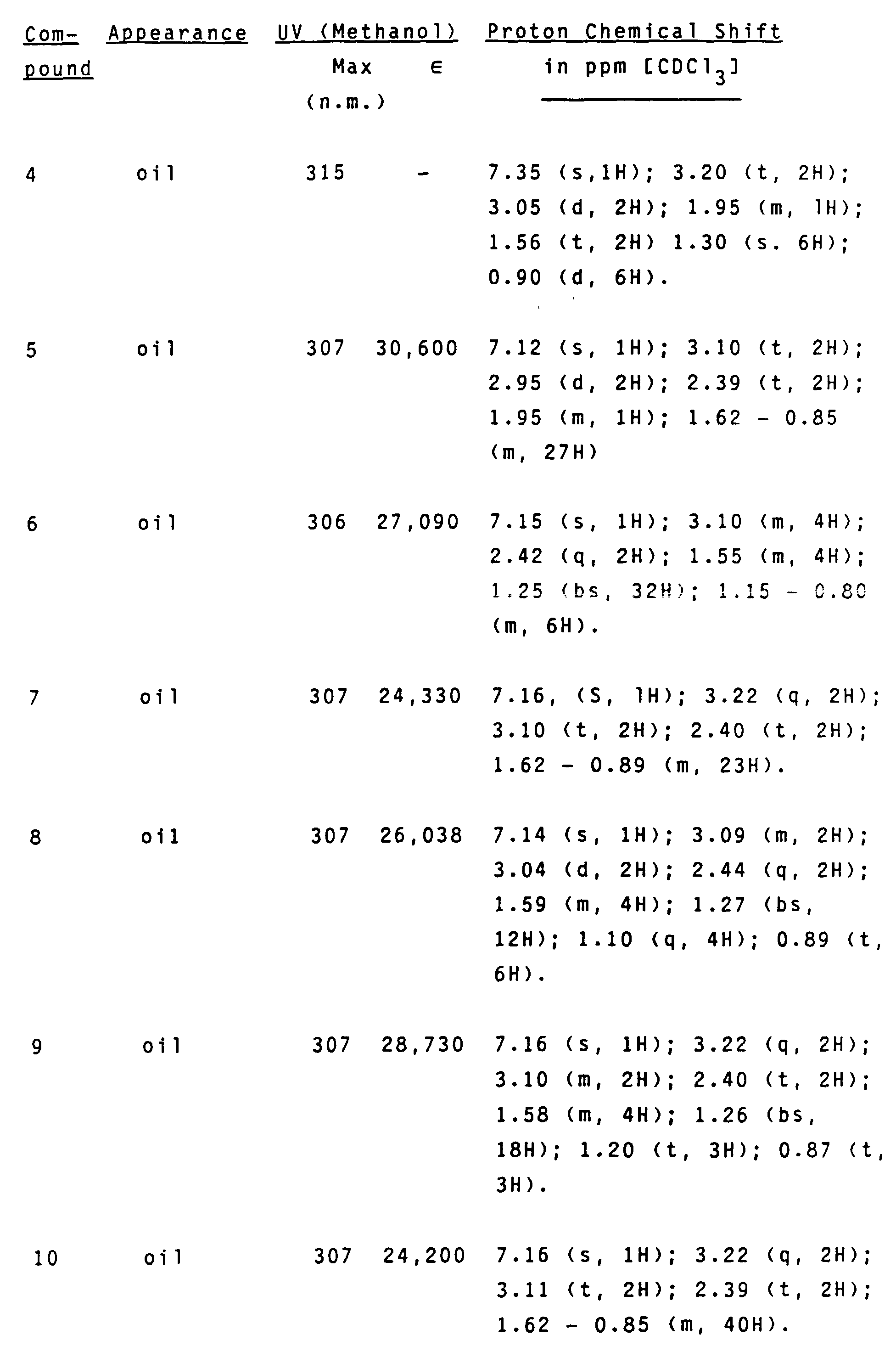

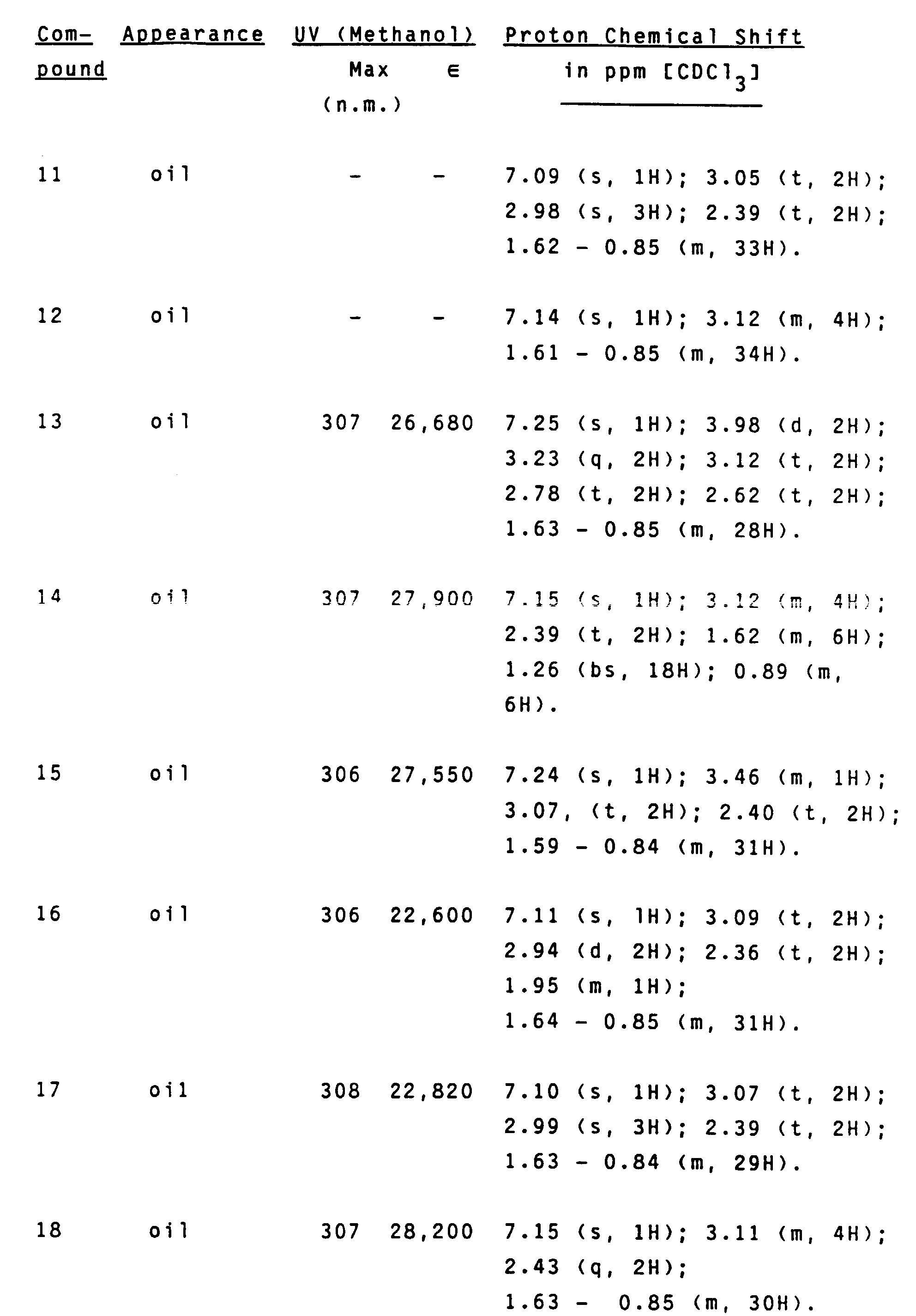

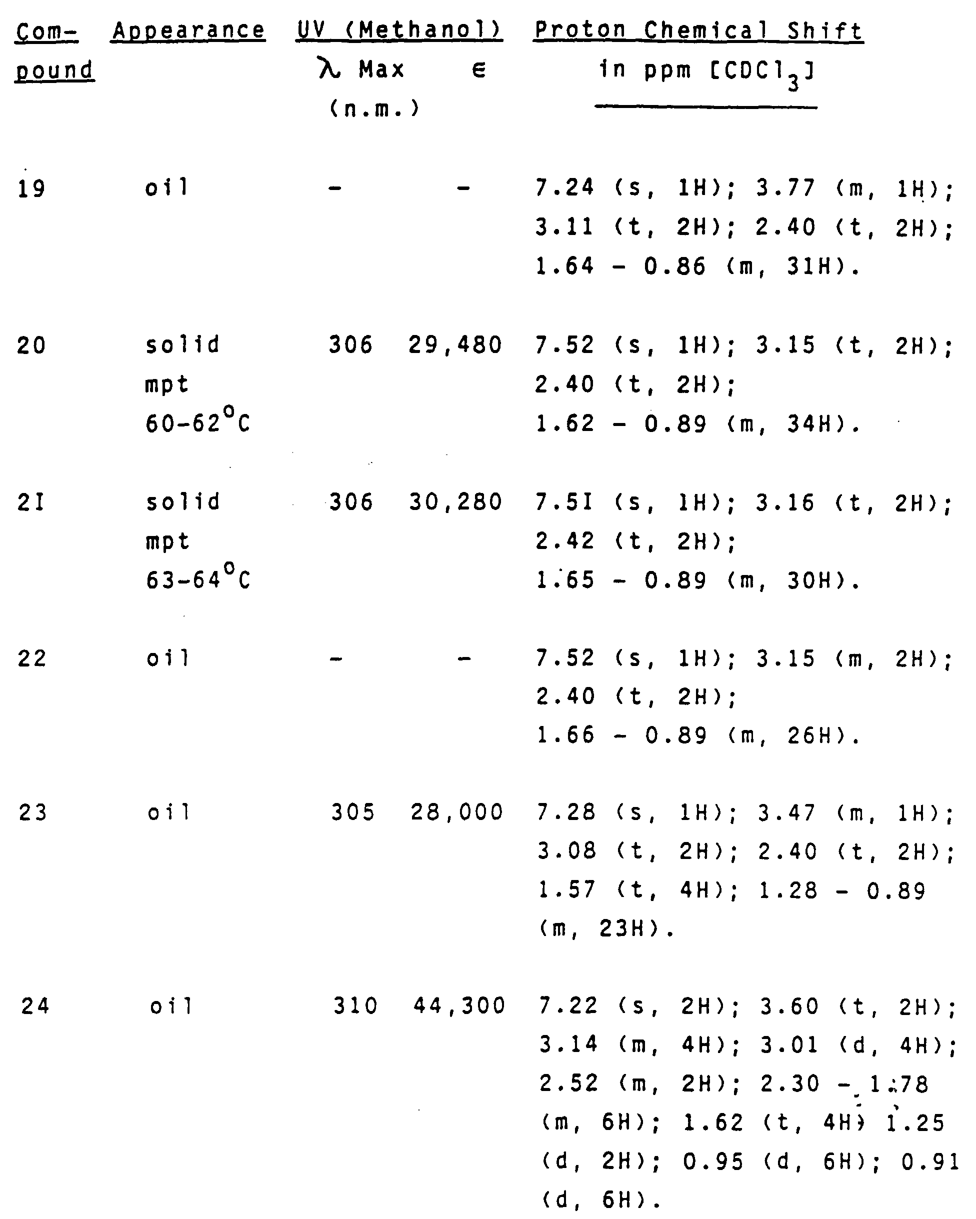

### Example 25

This example demonstrates an alternative procedure for preparation of compounds of the invention of formula VII The Compound 1-(2-methylpropyl)-4,4-dimethyl-1,4,5,6-tetrahydropyridine was prepared by the following procedure.
(a) N-(2-Methylpropyl)-3,3-dimethylglutarimide
   Isobutylamine (7.3g, 0.1 mol) was added slowly to 3,3-dimethylglutaric anhydride (14.2g, 0.1 mol) stirring at room temperature in a two neck round bottom flask fitted with a water condenser. After 30 minutes, acetic anhydride (20.4g, 0.2 mol) was added, and the resulting mixture was boiled for two hours. After cooling, the mixture was poured slowly on to an aqueous NaOH solution (2N, 100 ml) stirring at room temperature. After one hour, the mixture was diluted with diethyl ether (50ml), and the organic layer was separated. The aqueous layer was washed with more diethyl ether (2 x 50 ml), and the combined organic layer dried and concentrated to give the title compound as a pale yellow oil which solidified on standing, m.p. 48-52°C, yield 18.5g (94%). The product was suffuciently pure by NMR spectroscopy to be used directly in the following procedure.
(b) 1-(2-Methylpropyl)-4,4-dimethylpiperidine
   A solution of N-(2-Methylpropyl)-3,3-dimethylglutarimide (19.5g, 0.0984 mol) in dry ether (30 ml) was added via a dropping funnel over 30 minutes to a suspension of lithium aluminum hydride (5.36g, 0.141 mol) in dry ether (50 ml) at 0°C stirring under nitrogen. The mixture was then boiled for 3 hours, allowed to cool to room temperature and the excess lithium aluminium hydride was decomposed by careful addition of solid sodium sulphate decahydrate (20 g). After the suspended solid had turned white, the mixture was filtered, the solid washed twice with ether (2 x 50 ml), and the combined filtrate concentrated to yield a colourless liquid. Distillation gave the pure product, 1-(2-methylpropyl)-4,4-dimethylpipeidine, 13.0g (82%), b.p. 81-82°C/16mmHg. IH NMR (CDCl₃) δ 2.30 (4H, t), 2.05 (2H, d), 1.80 (lH, m), 1.35 (4H, t), 0.90 (6H, s) and 0.85 (6H, d).
(c) 1-(2-Methylpropyl)-4,4-dimethyl-1,4,5,6-tetrahydropyridine
   A solution of 1-(2-Methylpropyl)-4,4-dimethylpiperidine (13,0g, 0.077 mol) and mercuric acetate (98.8g, 0.31 mol, 4 molar equivalent) in 5% aqueous acetic acid (300ml) was boiled for 2.5 hours. On cooling, the white solid (mercurous acetate) was filtered-off, and the filtrate was treated repeatedly with hydrogen sulphide gas (3X), each time the mixture was filtered through a celite pad, the final filtrate made basic to pH 10 with K₂CO₃. The aqueous layer was extracted with ether (3 x 100 ml), and the combined organic layers dried over anhydrous MgSO₄, concentrated and distilled to give the product, 1-(2-methylpropyl)-4,4-dimethyl-1,4,5,6 -tetrahydropyridine, 7.1g (55%), b.p. 50 -53°C (2mmHg). 1HNMR (CDCl₃) δ 5.73 (IH, d), 4.05 (1H, d), 2.88 (2H, t), 2.55 (2H, d), 1.80 (1H, m), 1.55 (2H, t), 0.95 (6H, s), and 0.85 (6H, d).

### Example 26

This example demonstrates an alternative procedure for preparation of formula III which may be used in preparation of compounds of formula I.

The compound 5-bromo-3,3-dimethyl pentanal was prepared by the following procedure which may be used in place of the procedure of Example 1 Part (a) in preparing compounds of the invention such as for example compounds 1 to 32 inclusive.
(a) 3,3-Dimethylpent-4-enoic Acid
   To a solution of methyl 3,3-dimethylpent-4-enoate (0.5 mole) in 500 ml of ethanol (95% grade) was added potassium hydroxide (1 mole). The solution was refluxed for 1.5 hours. After cooling down to room temperature, the solution was concentrated to 200 ml on a rotary evaporator, and acidified by the addition of 5N H2SO4 solution. The product was isolated by extraction with diethyl ether (3 x 200 mls). After removal of the solvent, the crude product was distilled under reduced pressure to yield the pure acid (77% yield) b.p. 78 - 80°C (1.5mmHg). ¹H NMR (CDCl₃) δ 10.60 (1H, broad s), 5.90 (1H, dd, J 10.3, 17.6 Hz), 5.00 (1H, dd, J 17.6Hz, J 1.3Hz), 4.97 (1H, dd, J 10.3Hz, J 1.3Hz), 2.35 (2H, s), and 1.20 (6H, s).
(b) 5-Bromo-3,3-dimethylpentanoic Acid
   A stream of dry hydrogen bromide gas (made from 83.3 ml of tetralin and 71 ml of bromine) was bubbled through a stirred solution of 3,3-dimethylpent-4-enoic acid (80g, 0.625 mol) in dry light petroleum b.p. 40 - 60°C (940 ml) together with benzoyl peroxide (6.4g, 0.025 mol) until it was saturated (about 2 hours) whilst the reaction mixture was maintained at the temperature between 10 - 20°C by occasional cooling. After completion of the reaction, the mixture was cooled down to -10°C and the solid product was collected (near quantitative yield). The product was sufficiently pure by NMR spectroscopy to be used directly in the next step synthesis. However, a pure product was obtained after recrystallised from light petroleum spirit m.p. 53 - 55°C. ¹H NMR δ 3.43 (2H, t), 2.27 (2H, s), 2.10 (2H, t), and 1.08 (6H, s).
(c) 5-Bromo-3,3-dimethylpentanoyl chloride
   5-Bromo-3,3-dimethylpentanoic acid (40g, 0.192 mol) was converted into the corresponding acid chloride by heating it on a water bath with thionyl chloride (29.6g, 1.3 equivalents). The title compound was obtained by vacuum distillation as a colourless liquid, 38.8g (89% yield), b.p. 77°C/0.75 mm Hg. ¹H NMR (CDCl₃) δ 3.40 (2H, t), 2.85 (2H, s), 2.00 (2H, t), and 1.10 (6H, s).
(d) 5-Bromo-3,3-dimethylpentanal

The title compound was prepared by the Rosenmund reduction of 5-bromo-3,3-dimethylpentanoyl chloride using a similar procedure described in the literature (Burgstahler, Weigel and Shaefer, Synthesis, 1976, p. 767). The pure 5-bromo-3,3-dimethylpentanal was obtained as a colourless oil after distillation, b.p. 48 - 50°C (0.3mm Hg). ¹H NMR (CDCl₃) δ 9.77 (IH,t, J 2.0 Hz), 3.39 (2H, m), 2.32 (2H, d, J 2.0 Hz), 1.97 (2H, m), and 1.11 (6H, s).

In an alternative procedure the title compound was prepared by the Rosenmund reduction of 5-bromo-3,3-dimethylpentanoyl chloride using a similar procedure as described in Vogel, Practical Organic Chemistry, p. 765.

The experiment was designed and set-up as indicated in the above reference.

5-Bromo-3,3-dimethylpentanoyl chloride (16g, 0.070 mol) and 5% palladium-barium sulphate catalyst (0.8g, 5% w/w) were added to dry toluene (100 ml). A slow stream of dry hydrogen gas was passed through the stirred mixture heated at reflux. The course of the reaction was followed by the rate of hydrogen chloride evolution (titration with base) until approximately 85-90% of the theoretical amount of hydrogen chloride was generated. After approximately 3h, evolution of hydrogen chloride ceased. The reaction mixture was cooled and purged with nitrogen for 5 min. The catalyst was filtered off through a celite pad and filtrate concentrated invacu yielding 13.5g (100%) of the title compound. The material was anlaysed by G.C.

### Example 27

A coating composition may be prepared by blending compound 1 prepared according to Example 1 into a standard thermosetting acrylic/melamine-formaldehyde resin system at a rate of 2% by weight. The resulting composition is useful as a clearcoat in an automotive "base coat - clearcoat" coating system.

### Example 28

A sunscreen composition for topical application to skin may be prepared by mixing the following components in the specific proportion where the sunscreen compound is compound 1.

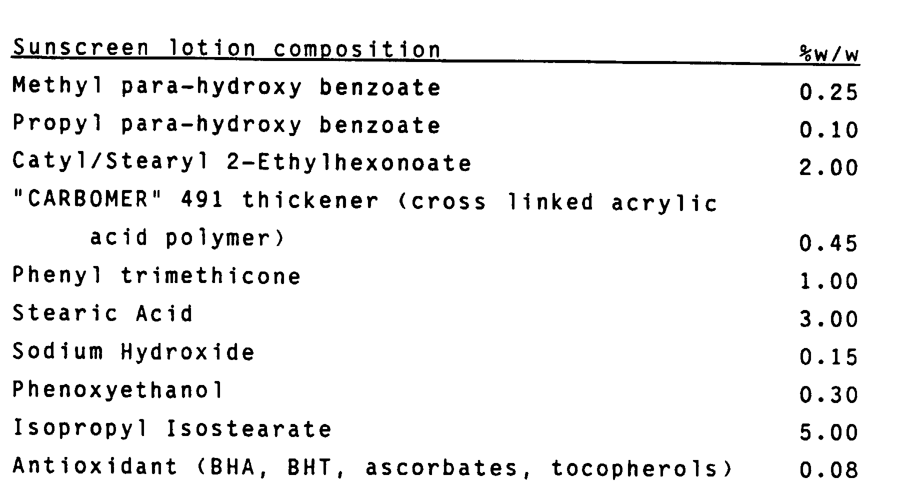

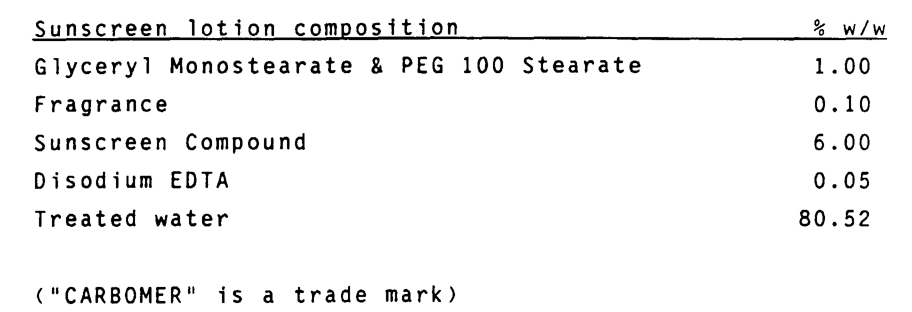

### Example 29

A plastics composition suitable for moulding was prepared by forming an intimate mixture of polypropylene GSM 109 (moulding grade) 0.1% w/w calcium stearate, 0.1% w/w antioxidant ("IRGANOX" B225, IRGANOX is a trade mark) and 0.1% w/w of compound 1 prepared according to Example 1.

### Example 30

A sunscreen composition for topical application to skin was prepared by mixing the following components where the sunscreen compound is compound 1.

| | |
|---|---|
| Water | 68.9 |
| Carbomer 941 | 0.2 |
| Isopropyl palmitate | 20.0 |
| Glyceryl stearate and PEG 100 stearate mixture | 5.0 |
| Polysorbate 60 | 5.0 |
| Sunscreen | 0.5 |
| BHT | 0.2 |
| Sodium thiosulphate | 0.2 |

The pH of the composition was adjusted to 7.

### Example 31

A sunscreen composition for topical application to skin may be prepared by mixing the following components where the sunscreen compound is compound 1.

| Sunscreen lotion composition | %w/w |
|---|---|
| Carbomer 934 | 0.65 |
| Ammonia (12.5% Solution) | 1.31 |
| Glyceryl stearate and PEG 100 stearate | 1.00 |
| Carnauba wax | 1.50 |
| Isostearic acid | 2.00 |
| Light liquid paraffin | 4.00 |
| Paraffin - soft white | 2.00 |
| Phenoxyethanol (and) methyl paraben (and) ethyl paraben (and) propyl paraben (and) butyl paraben | 0.80 |
| Dimethicone | 2.00 |
| Fragrance | 0.25 |
| Antioxidant (BHA, BHT, ascorbates, tocopherols) | 0.10 |
| Sunscreen compound | 10.00 |
| Microfine titanium dioxide | 5.00 |
| Water | 69.39 |

### Example 32

This example compares the ultraviolet light absorbing properties and water resistance properties of compounds of the invention.

E₁% is a relative measure of the UV absorbance of a 1% w/w solution of the compound.

For each compound in Table 6 below the log P was determined by the HPLC method of Leahy, Taylor and Wait Quant. Struct. Act Relat., 8, 17-31 (1989) and the results are recorded in Table 6.

| Compound No. | No. Carbon Atoms | E₁% | log P |
|---|---|---|---|
| 1 | 14 | 131 | 3.2 |
| 2 | 13 | 140 | 2.7 |
| 3 | 15 | 124 | 3.7 |
| 4 | 13 | 112 | 3.0 |
| 5 | 19 | 100 | 5.8 |
| 6 | 26 | 75 | 9.4 |
| 7 | 17 | 110 | 4.8 |
| 8 | 18 | 105 | 5.3 |
| 9 | 19 | 100 | 5.8 |
| 10 | 25 | 77 | 8.9 |
| 11 | 22 | 87 | 7.4 |
| 12 | 22 | 87 | 7.4 |
| 13 | 21 | 87 | 6.9 |
| 14 | 20 | 95 | 6.3 |
| 15 | 20 | 95 | 6.3 |
| 16 | 21 | 91 | 6.9 |
| 17 | 20 | 95 | 6.3 |
| 18 | 20 | 95 | 6.3 |
| 19 | 21 | 91 | 6.9 |
| 20 | 21 | 91 | 6.9 |
| 21 | 19 | 100 | 5.8 |
| 22 | 17 | 110 | 4.8 |
| 23 | 18 | 105 | 5.3 |
| 24 | 28 | 131 | 9.4 |

## Claims

1. A compound of formula I 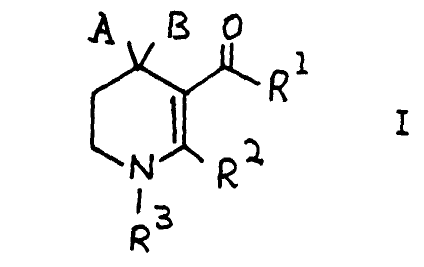 wherein
A and B are independently selected from C₁ to C₆ alkyl and C₁ to C₆ haloalkyl and wherein A and B may together form a carbocyclic ring of from 3 to 6 ring members;
R¹ is selected from the group consisting of C₁ to C₁₈ alkyl; C₁ to C₆ haloalkyl; C₁ to C₁₂ alkyl substituted with a substituent selected from hydroxy, amino, C₁ to C₁₆ alkoxy, C₁ to C₁₆ alkanoyl, (C₁ to C₁₆ alkoxy) carbonyl, phenyl and the radical of formula I(a) 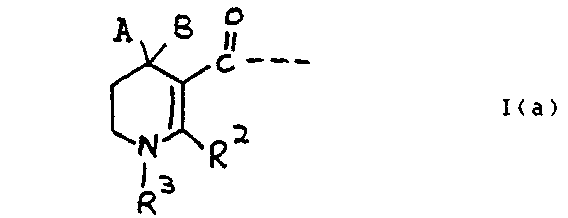 wherein A, B, R² and R³ are as herein defined; C₂ to C₁₈ alkenyl; C₅ to C₇ cycloalkyl; phenyl; the groups substituted phenyl and substituted benzyl wherein the substituent is a benzene ring substituent and is selected from the group consisting of C₁ to C₉ alkyl, C₂ to C₉ alkenyl and C₁ to C₉ alkoxy; R₂ is selected from the group consisting of hydrogen and C₁ to C₄ alkyl;
R³ is selected from the group consisting of C₁ to C₁₈ alkyl; C₁ to C₁₂ alkyl substituted with a substituent selected from the group consisting of hydroxy, amino, C₁ to C₁₆ alkoxy, phenyl and C₁ to C₁₆ alkanoyl; C₂ to C₁₈ alkenyl; C₅ to C₇ cycloalkyl; and substituted benzyl wherein the benzene ring is substituted with a substituent selected from the group consisting of C₁ to C₉ alkyl, C₂ to C₉ alkenyl and C₁ to C₉ alkoxy;

2. A compound according to claim 1 wherein;
A and B are independently selected from C₁ to C₆ alkyl;
R¹ is selected from the group consisting of C₁ to C₁₈ alkyl, C₁ to C₆ haloalkyl and C₁ to C₆ alkyl substituted by a substituent selected from the group consisting of C₁ to C₉ alkoxy, C₁ to C₉ alkanoyl, (C₁ to C₉ alkoxy) carbonyl and the radical of formula I(a) wherein A, B, R¹, R² and R³ are as formula I(a) wherein A, B, R¹, R² and R³ are as herein defined;
R² is hydrogen; and
R³ is selected from the group consisting of C₁ to C₁₈ alkyl.

3. A compound according to claim 1 wherein:
A and B are methyl;
R¹ is selected from C₁ to C₁₈ alkyl;
R² is hydrogen; and
R³ is C₂ to C₁₈ alkyl.

4. A compound according to claim 1 wherein one of the groups R¹ and R³ is selected from C₁ to C₆ alkyl and the other is selected from C₄ to C₁₈ alkyl.

5. A compound according to claim 4 wherein one of R¹ and R³ is selected from C₁ to C₄ alkyl and the other is selected from C₆ to C₁₈ alkyl.

6. A compound according to claim 3 which comprises from 14 to 31 carbon atoms.

7. A compound according to claim 2 wherein log P of said compound is in the range of from 3.2 to 10.9.

8. A process for preparation of a compound of formula I according to claim 1 which-process comprises reacting a compound of formula VII with an acylating agent of formula VIII or IX.

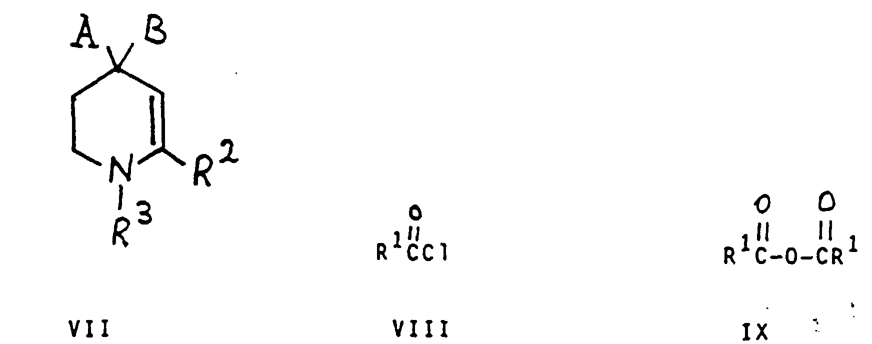

9. A process according to claim 8 wherein the compound of formula VII is prepared i) by reaction of an amine of formula II with a compound of formula III in order to obtain a compound of formula VII wherein R² is hydrogen; or ii) by reacting an amine of formula II with an α-pyrone of formula IV to provide an α-pyridone of formula V and then reducing said α-pyridone of formula V 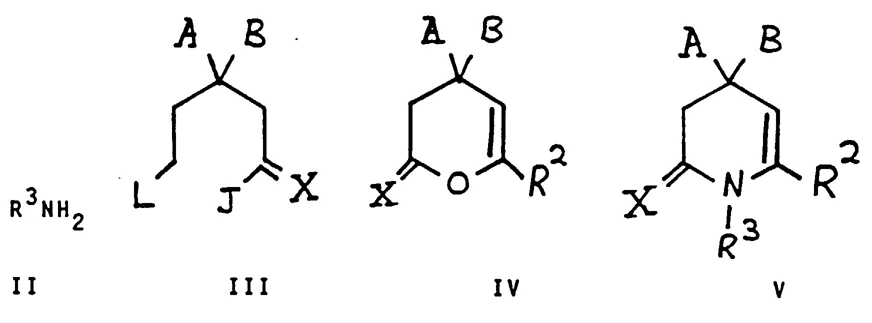 wherein in the compounds of formula III, IV and V the group X is oxo or an acetal derivative thereof and in the compound of formula III J is selected from the group of hydrogen, hydroxy, chloro and C₁ to C₆ alkoxy and L is a leaving group.

10. A process according to claim 9 wherein a compound of formula VII in which R² is hydrogen is prepared by reaction of a compound of formula II with a compound of formula III wherein L is a leaving group and X is oxo or an acetal derivative thereof said acetal being selected from di-(C₁ to C₄ alkoxy), ethylenedioxy propylenedioxy and 2,2-dimethylpropylenedioxy and wherein when X is oxo J is selected from hydrogen, hydroxy, chloro and C₁ to C₆ alkoxy and wherein when X is an acetal derivative then J is hydrogen.

11. An intermediate compound of formula VII 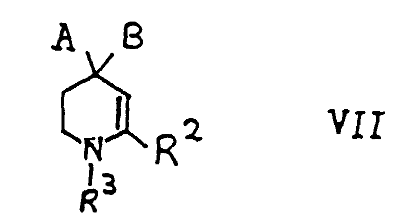 wherein A, B, R² and R³ are as defined according to claim 3.

12. A sunscreen composition comprising an effective amount of a compound of formula I defined according to claim 1 and a carrier therefor.

13. A sunscreen composition according to claim 12 comprising in the range of from 0.5 to 30% by weight of the compound of formula I.

14. A coating composition such as paint comprising in the range of from 0.001 to 5% w/w of the compound of formula I according to claim 1.

15. A plastics composition comprising a polymeric component and in the range of from 0.01 to 45% w/w of a compound of formula I defined according to claim 1.

16. A process for protecting a surface from ultra-violet radiation comprising applying to said surface a sunscreen composition according to claim 12.

## Patentansprüche

1. Eine Verbindung der Formel I 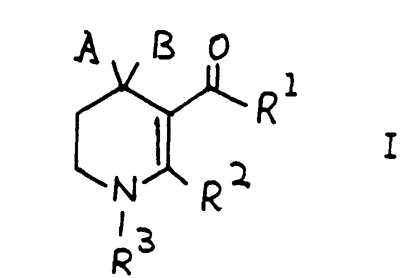 in der
A und B unabhängig voneinander ausgewählt sind aus C₁ bis C₆-Alkyl und C₁ bis C₆-Halogenalkyl, und worin A und B zusammen einen carbocyclischen Ring aus 3 bis 6 Ringgliedern bilden können;
R¹ ausgewählt ist aus der Gruppe, die besteht aus C₁ bis C₁₈-Alkyl; C₁ bis C₆-Halogenalkyl; C₁ bis C₁₂-Alkyl, das mit einem Substituenten substituiert ist, der ausgewählt ist aus Hydroxy, Amino, C₁ bis C₁₆-Alkoxy, C₁ bis C₁₆-Alkanoyl, (C₁ bis C₁₆-Alkoxy)carbonyl, Phenyl und dem Rest der Formel I(a) 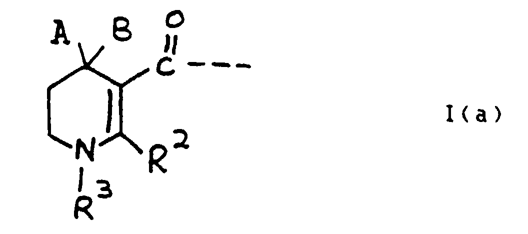 in der A, B, R² und R³ wie hierin angegeben definiert sind; C₂ bis C₁₈-Alkenyl; C₅ bis C₇-Cycloalkyl; Phenyl; den Gruppen der substituierten Phenyl- und substituierten Benzylreste, worin der Substituent ein Substituent am Benzolring ist und aus der Gruppe ausgewählt ist, die aus C₁ bis C₉-Alkyl, C₂ bis C₉-Alkenyl und C₁ bis C₉-Alkoxy besteht;
R² ausgewählt ist aus der Gruppe, die aus Wasserstoff und C₁ bis C₄-Alkyl besteht;
R³ ausgewählt ist aus der Gruppe, die aus C₁ bis C₁₈-Alkyl; C₁ bis C₁₂-Alkyl, das substituiert ist mit einem Substituenten, der ausgewählt ist aus der Gruppe, die besteht aus Hydroxy, Amino, C₁ bis C₁₆-Alkoxy, Phenyl und C₁ bis C₁₆-Alkanoyl; C₂ bis C₁₈-Alkenyl; C₅ bis C₇-Cycloalkyl; sowie substiutiertem Benzyl, worin der Benzolring mit einem Substituenten substituiert ist, der aus der Gruppe ausgewählt ist, die besteht aus C₁ bis C₉-Alkyl, C₂ bis C₉-Alkenyl und C₁ bis C₉-Alkoxy.

2. Eine Verbindung nach Anspruch 1, worin:
A und B unabhängig voneinander ausgewählt sind aus C₁ bis C₆-Alkyl;
R¹ ausgewählt ist aus der Gruppe, die besteht aus C₁ bis C₁₈-Alkyl; C₁ bis C₆-Halogenalkyl und C₁ bis C₆-Alkyl, das substituiert ist durch einen Substituenten, der ausgewählt ist aus der Gruppe, die besteht aus C₁ bis C₉-Alkoxy, C₁ bis C₉-Alkanoyl (C₁ bis C₉-Alkoxy)carbonyl und dem Rest der Formel I(a), worin A, B, R¹, R² und R³ wie hierin angegeben definiert sind;
R² Wasserstoff ist; und
R³ ausgewählt ist aus der Gruppe, die aus C₁ bis C₁₈-Alkyl besteht.

3. Eine Verbindung nach Anspruch 1, worin:
A und B Methyl sind;
R¹ ausgewählt ist aus C₁ bis C₁₈-Alkyl;
R² Wasserstoff ist; und
R³ C₂ bis C₁₈-Alkyl ist.

4. Eine Verbindung nach Anspruch 1, worin eine der Gruppen R¹ und R³ ausgewählt ist aus C₁ bis C₆-Alkyl und die andere ausgewählt ist aus C₄ bis C₁₈-Alkyl.

5. Eine Verbindung nach Anspruch 4, worin eine von R¹ und R³ ausgewählt ist aus C₁ bis C₄-Alkyl und die andere ausgewählt ist aus C₆ bis C₁₈-Alkyl.

6. Eine Verbindung nach Anspruch 3, die von 14 bis 31 Kohlenstoffatome enthält.

7. Eine Verbindung nach Anspruch 2, worin log P der genannten Verbindung im Bereich von 3,2 bis 10,9 liegt.

8. Verfahren zur Herstellung einer Verbindung der Formel I von Anspruch 1, wobei das Verfahren die Umsetzung einer Verbindung der Formel VII mit einem Acylierungsmittel der Formel VIII oder IX umfaßt.

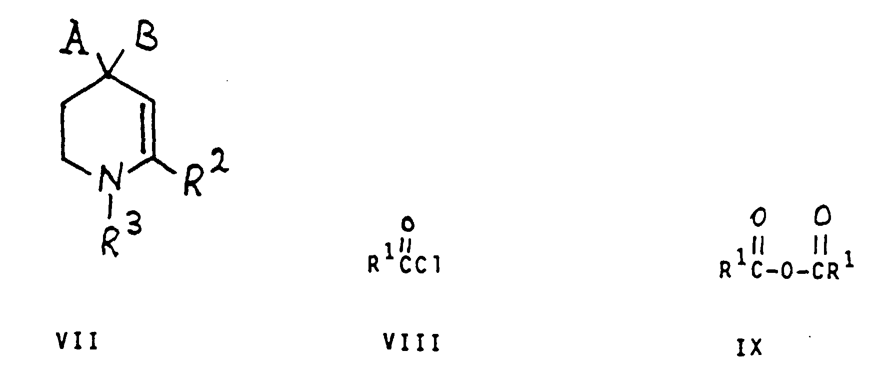

9. Verfahren nach Anspruch 8, bei dem die Verbindung der Formel VII hergestellt wird i) durch Umsetzung eines Amins der Formel II mit einer Verbindung der Formel III, um eine Verbindung der Formel VII zu erhalten, in der R² Wasserstoff ist; oder ii) durch Umsetzung eines Amins der Formel II mit einem α-Pyron der Formel IV, um ein α-Pyridon der Formel V zu erhalten, und anschließendes Reduzieren des genannten α-Pyridons der Formel V, 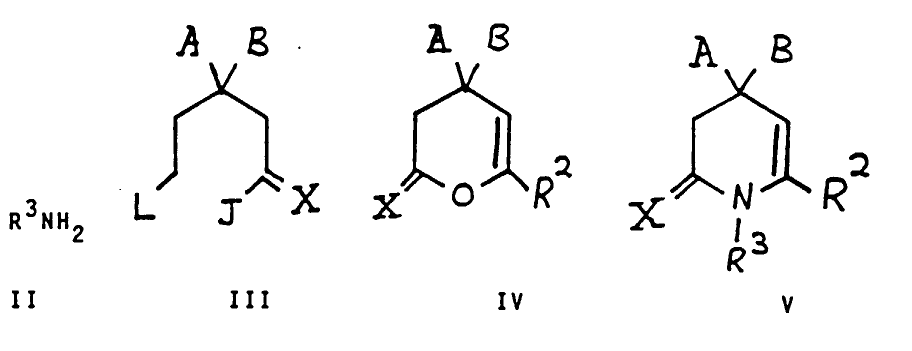 worin in den Verbindungen der Formeln III, IV und V die Gruppe X eine Oxogruppe oder ein Acetalderivat davon ist und in der Verbindung der Formel III J ausgewählt ist aus der Gruppe Wasserstoff, Hydroxy, Chlor und C₁ bis C₆-Alkoxy und worin L eine austretende Gruppe ist.

10. Verfahren nach Anspruch 9, bei dem eine Verbindung der Formel VII, in der R² Wasserstoff ist, dadurch hergestellt wird, daß man eine Verbindung der Formel II mit einer Verbindung der Formel III umsetzt, in der L eine austretende Gruppe ist und X für eine Oxogruppe oder ein Acetalderivat davon steht, wobei das genannte Acetal ausgewählt ist aus Di-(C₁ bis C₄-Alkoxy), Ethylendioxy, Propylendioxy und 2,2-Dimethylpropylendioxy, und worin dann, wenn X für eine Oxogruppe steht, J ausgewählt ist aus Wasserstoff, Hydroxy, Chlor und C₁ bis C₆-Alkoxy, und worin dann, wenn X ein Actalderivat ist, J Wasserstoff ist.

11. Eine Zwischenstufen-Verbindung der Formel VII 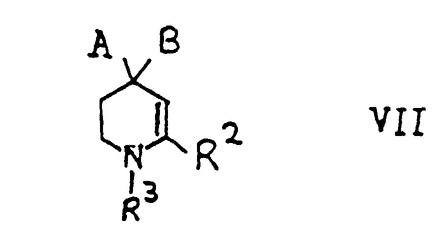 worin A, B, R² und R³ wie in Anspruch 3 definiert sind.

12. Eine Sonnenschutzzusammensetzung, die eine wirksame Menge einer Verbindung der Formel I, wie sie gemäß Anspruch 1 definiert ist, sowie einen Träger dafür umfaßt.

13. Eine Sonnenschutzzusammensetzung nach Anspruch 12, die die Verbindung der Formel I im Bereich von 0,5 bis 30 Gew.% aufweist.

14. Eine Überzugszusammensetzung wie beispielsweise eine Farbe, die die Verbindung der Formel I nach Anspruch 1 im Bereich von 0,01 bis 5 Gew.% (Gewicht/Gewicht) enthält.

15. Eine Kunststoffzusammensetzung mit einer Polymerkomponente sowie einer Verbindung der Formel I, wie sie gemäß Anspruch 1 definiert ist, im Bereich von 0,01 bis 45 Gew.% (Gewicht/Gewicht).

16. Verfahren zum Schützen einer Oberfläche gegen Ultraviolettlichtstrahlung, das das Aufbringen einer Sonnenschutzzusammensetzung nach Anspruch 12 auf die genannte Oberfläche umfaßt.

## Revendications

1. Composé de formule I 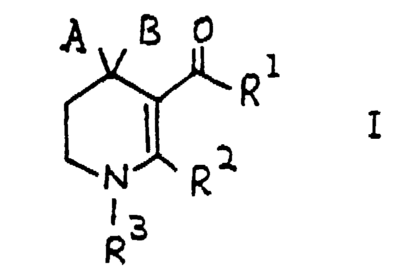 dans laquelle
A et B sont choisis indépendamment parmi un alkyle en C₁ à C₆ et un haloalkyle en C₁ à C₆, et dans laquelle A et B peuvent former ensemble un noyau carbocyclique à 3 à 6 maillons;
R¹ est choisi dans le groupe comprenant un alkyle en C₁ à C₁₈; un haloalkyle en C₁ à C₆; un alkyle en C₁ à C₁₂ substitué par un substituant choisi parmi un radical hydroxy, amino, alkoxy en C₁ à C₁₆, alcanoyle en C₁ à C₁₆, (alkoxy en C₁ à C₁₆)carbonyle, phényle et le radical de formule (I)a 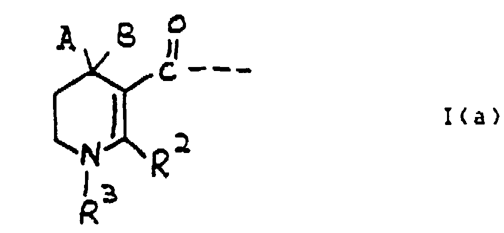 dans laquelle A, B, R² et R³ sont tels que définis ici; un alcényle en C₂ à C₁₈; un cylcoalkyle en C₅ à C₇; un phényle; les radicaux phényles substitués et benzyles substitués dans lesquels le substituant est un substituant du noyau benzène et est choisi dans le groupe comprenant un alkyle en C₁ à C₉, un alcényle en C₂ à C₉ et un alkoxy en C₁ à C₉; R₂ est choisi dans le groupe comprenant l'hydrogène et un alkyle en C₁ à C₄;
R³ est choisi dans le groupe comprenant un alkyle en C₁ à C₁₈; un alkyle en C₁ à C₁₂ substitué par un substituant choisi dans le groupe comprenant un radical hydroxy, amino, alkoxy en C₁ à C₁₆, phényle et alcanoyle en C₁ à C₁₆; alcényle en C₂ à C₁₈; cycloalkyle en C₅ à C₇; et benzyle substitué dans lequel le noyau benzène est substitué par un substituant choisi dans le groupe comprenant un alkyle en C₁ à C₉, un alcényle en C₂ à C₉ et un alkoxy en C₁ à C₉;

2. Composé selon la revendication 1 dans lequel: A et B sont choisis indépendamment parmi un alkyle en C₁ à C₆;
R¹ est choisi dans le groupe comprenant un alkyle en C₁ à C₁₈, un haloalkyle en C₁ à C₆ et un alkyle en C₁ à C₆ substitué par un substituant choisi dans le groupe comprenant un alkoxy en C₁ à C₉, alcanoyle en C₁ à C₉, un (alkoxy en C₁ à C₉)carbonyle et le radical de formule I(a) dans laquelle A, B, R¹, R² et R³ sont définis ici;
R² est de l'hydrogène; et
R³ est choisi parmi le groupe comprenant un alkyle en C₁ a C₁₈.

3. Composé selon la revendication 1 dans lequel:
A et B représentent le méthyle;
R¹ est choisi parmi les alkyles en C₁ à C₁₈;
R² est l'hydrogène; et
R³ est un alkyle en C₂ à C₁₈,

4. Composé selon la revendication 1 dans lequel l'un des radicaux R¹ et R³ est choisi parmi les alkyles C₁ à C₆, et l'autre est choisi parmi les alkyles en C₄ à C₁₈.

5. Composé selon la revendication 4 dans lequel l'un de R¹ et R³ est choisi parmi les alkyles C₁ à C₄ et l'autre est choisi parmi les alkyles en C₆ à C₁₈.

6. Composé selon la revendication 3 comprenant 14 à 31 atomes de carbone.

7. Composé selon la revendication 2 dans lequel log P dudit composé se situe dans l'intervalle de 3,2 à 10,9.

8. Procédé de préparation d'un composé de formule I selon la revendication 1, lequel procédé consiste à faire réagir un composé de formule VII avec un agent d'acylation de formule VIII ou IX.

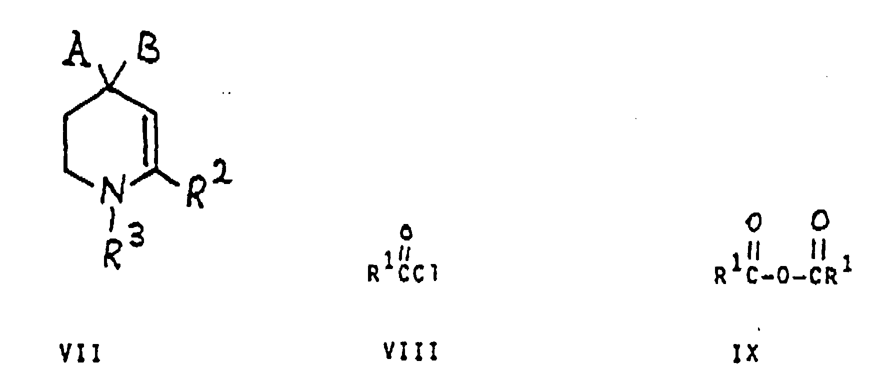

9. Procédé selon la revendication 8 dans lequel le composé de formule VII est préparé i) par réaction d'une amine de formule II avec un composé de formule III afin d'obtenir un composé de formule VII dans lequel R² est l'hydrogène; ou ii) par réaction d'une amine de formule II avec une α-pyrone de formule IV pour fournir une α-pyridone de formule V et ensuite réduction de ladite α-pyridone de formule V 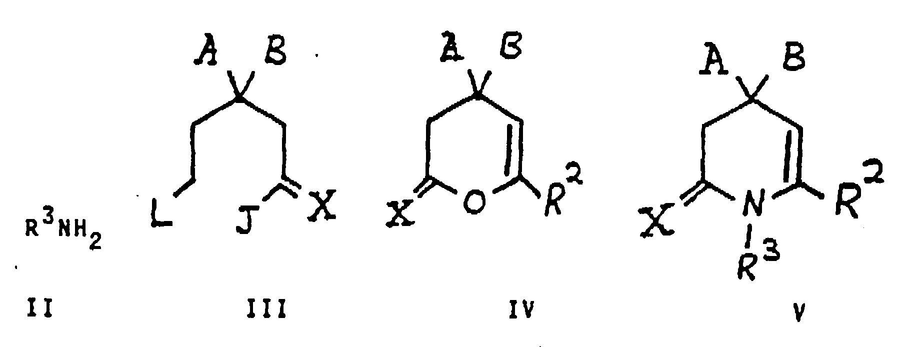 dans lesquelles, dans les composés de formule III, IV et V, le groupe X est un oxo ou un dérivé d'acétal de celui-ci, et dans le composé de formule III J est choisi dans le groupe de l'hydrogène, hydroxy, chloro et alkoxy en C₁ à C₆ et L est un groupe qui s'élimine.

10. Procédé selon la revendication 9 dans lequel un composé de formule VII, dans laquelle R² est l'hydrogène, est préparé en faisant réagir un composé de formule II avec un composé de formule III dans laquelle L est un groupe qui s'élimine et X est un oxo ou un dérivé d'acétal de celui-ci, ledit acétal étant choisi parmi di-(alkoxy en C₁ à C₄), éthylènedioxy, propylènedioxy et 2,2-diméthylpropylènedioxy et dans laquelle, lorsque X est un oxo, J est choisi parmi hydrogène, hydroxy, chloro et alkoxy en C₁ à C₆ et dans laquelle, lorsque X est un dérivé d'acétal, J est l'hydrogène.

11. Composé intermédiaire de formule VII 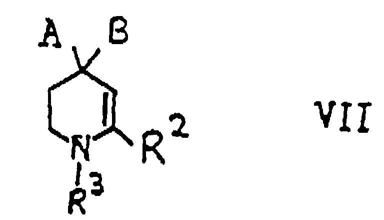 dans laquelle A, B, R² et R³ sont tels que définis à la revendication 3.

12. Composition d'écran solaire comprenant une quantité efficace d'un composé de formule I défini selon la revendication 1, et un support pour celui-ci.

13. Composition d'écran solaire selon la revendication 12 comprenant le composé de formule I dans un intervalle de 0,5 à 30% en poids.

14. Composition de revêtement telle qu'une peinture comprenant le composé de formule I selon la revendication 1 dans un intervalle de 0,001 à 5% en poids.

15. Composition de matière plastique comprenant un composant polymérique et un composé de formule I défini selon la revendication 1 dans un intervalle de 0,01 à 45% en poids.

16. Procédé de protection d'une surface vis-à-vis d'un rayonnement ultra-violet comprenant l'application sur ladite surface d'une composition d'écran solaire selon la revendication 12.
